**Europäisches Patentamt**

**European Patent Office**

**Office européen des brevets**

(19)

(11) Veröffentlichungsnummer : **0 394 819 B1**

(12) # EUROPÄISCHE PATENTSCHRIFT

(45) Veröffentlichungstag der Patentschrift :
**18.01.95 Patentblatt 95/03**

(51) Int. Cl.⁶ : **G01N 33/537,** G01N 33/577, C12P 21/08, C12N 5/12

(21) Anmeldenummer : **90107323.9**

(22) Anmeldetag : **18.04.90**

(54) **Spezifische Antikörper gegen Troponin T, ihre Herstellung und Verwendung in einem Reagenz zur Bestimmung von Herzmuskelnekrosen.**

(30) Priorität : **25.04.89 DE 3913568**
**12.07.89 DE 3922873**

(43) Veröffentlichungstag der Anmeldung :
**31.10.90 Patentblatt 90/44**

(45) Bekanntmachung des Hinweises auf die Patenterteilung :
**18.01.95 Patentblatt 95/03**

(84) Benannte Vertragsstaaten :
**AT BE CH DE DK ES FR GB GR IT LI LU NL SE**

(56) Entgegenhaltungen :
**EP-A- 0 269 092**
**JOURNAL OF MOLECULAR AND CELLULAR CARDIOLOGY, Band 19, Nr. 7, Juli 1987; B. CUMMINS et al., Seiten 999-1010**
**CHEMICAL ABSTRACTS, Band 107, Nr. 9, 31 August 1987, Columbus, OH (US); Y. OGASA-WARA et al., Seite 430, Nr. 74686c**

(56) Entgegenhaltungen :
**CHEMICAL ABSTRACTS, Band 103, Nr. 13, 30 September 1985, Columbus, OH (US); I.M. BIRD et al., Seite 383, Nr. 102276g**
**CHEMICAL ABSTRACTS, Band 102, Nr. 15, 15 April 1985, Columbus, OH (US); I.M.BIRD et al., Seite 389, Nr. 129296e**
**CHEMICAL ABSTRACTS, Band 102, Nr. 7, 18 Februar 1985, Columbus, OH (US); S.S. LIM et al., Seite 452, Nr. 60465q**

(73) Patentinhaber : **BOEHRINGER MANNHEIM GMBH**
**D-68298 Mannheim (DE)**

(72) Erfinder : **Katus, Hugo, Prof. Dr.**
**Lachenweg 6**
**D-6901 Bammental (DE)**
Erfinder : **Borgya, Anneliese**
**Kustermannstrasse 3**
**D-8132 Tutzing (DE)**
Erfinder : **Hallermayer, Klaus, Dr.**
**Barthstrasse 31**
**D-8000 München 2 (DE)**
Erfinder : **Looser, Siegfried, Dr.**
**Ammerstrasse 27**
**D-8120 Weilheim (DE)**

**EP 0 394 819 B1**

**Beschreibung**

Die Erfindung betrifft spezifische Antikörper gegen Herzmuskel-Troponin-T, deren Herstellung und Verwendung in einem immunologischen Reagenz zur Bestimmung von Herzmuskelnekrosen.

Die Myofibrillen des quergestreiften Muskels bestehen aus zwei zusammenwirkenden Proteinfilamenten, den dicken Filamenten mit Myosin als Hauptbestandteil und den dünnen Filamenten, die Actin, Tropomyosin und Troponine enthalten. Troponin ist in den Zellen als regulatives Strukturprotein zu einem Komplex zusammengelagert und besteht aus drei verschiedenen Proteinen:

Troponin C (Mg 18000) bindet Calciumionen

Troponin I (Mg 24000) eine actinbindende Untereinheit

Troponin T (Mg 37000) lagert sich an Tropomyosin an.

Die vergleichbare Nomenklatur hat historische Gründe, da ursprünglich der Komplex als solcher gereinigt und als ein einziges Protein angesehen und mit Troponin bezeichnet wurde. Erst später konnte nachgewiesen werden, daß Troponin in Wirklichkeit aus drei verschiedenen Proteinen besteht. Diese Nomenklatur wurde dann wegen der räumlichen Beziehung der Proteine auf dem dünnen Filament des kontraktilen Apparates und wegen ihrer Kooperation im Rahmen der Regulation der Muskelkontraktion beibehalten. Es handelt sich dabei jedoch um drei verschiedene Proteine, die mit den anderen Proteinen des kontraktilen Apparates, wie Myosin oder Actin, funktionell in Beziehung stehen, aber unterschiedliche Aminosäuresequenzen haben.

Bei lang dauernder schwerer Ischämie bzw. Muskelzellennekrosen gelangt Troponin I in das Blutplasma und stellt somit einen Parameter für derartige Erkrankungen dar, der auch zur Diagnose und Verlaufskontrolle, analog den bekannten "Infarktenzymen" CK, CK-MB, GOT und LDH eingesetzt werden kann.

Es hat sich gezeigt, daß die Bestimmung der CK und CK-MB nicht völlig spezifisch für einen Infarkt und nur 80 - 90 Stunden nach dem Infarkt im Serum erhöht ist. Auch GOT und LDH sind nicht herzmuskelspezifisch, da bei vielen anderen Erkrankungen ebenfalls erhöhte Werte im Blut gefunden werden.

Der Nachteil einer Bestimmung von kardialem Troponin I ist, daß im Serum normalerweise bereits Konzentrationen von Troponin I in der Höhe von 10 ng/ml auftreten (vgl. B. Cummins, J. Mol. Cell. Card. 19 (1987), 999 - 1010 und B. Cummins, Clin. Invest. 113 (1987) 1333 - 1344). Es zeigt sich, daß beim transmuralen Infarkt eine biphasische Serumkonzentration auftritt. Im Mittel war Troponin I von der 4. bis zur 168. Stunde nach Schmerzbeginn bei 37 Patienten mit akutem transmuralen Infarkt erhöht. Ähnliche Ergebnisse wurden im Tiermodell erhalten. Danach ergibt sich als Zeitintervall für die absolute diagnostische Sensitivität für Troponin I die 10. - 50. Stunde nach dem Infarktereignis. Somit kann neben der begrenzten Sensitivität wegen stark schwankender Serumwerte von Troponin I auch die klinisch wichtige Verlaufskontrolle über 1O Tage und länger nach dem Infarktereignis mit der Troponin I-Bestimmung nicht erreicht werden.

Die Aufgabe der vorliegenden Erfindung lag darin, diese Nachteile zu beseitigen und ein Bestimmungsverfahren zur Verfügung zu stellen, mit dem bei Myokardinfarkten und sonstigen Herzmuskelschädigungen eine Verlaufskontrolle über mindestens 150 Stunden (Dauer der absoluten diagnostischen Sensitivität) möglich ist

Diese Aufgabe wird gelöst durch ein Verfahren zur Bestimmung von Herzmuskelnekrosen nach dem Prinzip des Immunoassays, welches dadurch gekennzeichnet ist, daß eine Serum- oder Plasmaprobe mit mindestens einem Antikörper gegen Troponin T und einem Bindepartner B für Troponin T oder für den Antikörper inkubiert wird, wobei entweder der Antikörper gegen Troponin T oder der Bindepartner B mit einer bestimmbaren Gruppe markiert ist, der dabei gebildete immunologische Komplex, der die bestimmbare Gruppe enthält, abgetrennt und die bestimmbare Gruppe in der abgetrennten oder noch verbliebenen Phase als Maß für Troponin T aus der Probe bestimmt wird.

Der Bindepartner B muß an dem Antikörper gegen Troponin T oder an Troponin T binden. B kann beispielsweise ein zweiter Antikörper gegen Troponin T oder humanes oder tierisches Troponin T oder ein Analoges davon, welches von dem Antikörper gebunden wird, sein.

Vorzugsweise wird die Probe mit einem Antikörper gegen Troponin T und einem Konjugat aus einem weiteren Antikörper gegen Troponin T und einer bestimmbaren Gruppe inkubiert, der gebildete immunologische Komplex durch Phasentrennung abgetrennt und die bestimmbare Gruppe in einer der Phasen bestimmt.

Weiter bevorzugt ist es, die Probe mit einem Antikörper gegen Troponin T und einem Konjugat aus Troponin T und einer bestimmbaren Gruppe zu inkubieren, den gebildeten immunologischen Komplex durch Phasentrennung abzutrennen und die bestimmbare Gruppe in einer der Phasen zu bestimmen.

Es hat sich überraschenderweise gezeigt, daß durch einen Troponin T-Immunoassay eine deutlich höhere Sensitivität bei der Bestimmung von Herzmuskelnekrosen (wie z. B. durch Herzinfarkt, Ischämie oder Angina pectoris) als bei der Bestimmung anderer Parameter, wie CK, CK-MB, GOT, LDH oder Troponin I, erhalten wird. Wie die Erfinder festgestellt haben, ist dies dadurch begründet, daß im Gegensatz zu anderen Proteinen des kontraktilen Apparates für Troponin T bis zur Nachweisgrenze des Tests (0,25 ng/ml) kein Serumspiegel gemessen werden kann.

Dies ist besonders überraschend, da auf Grund der funktionellen Beziehung zwischen den Troponinen für Troponin T ein ähnlicher Serumspiegel wie für Troponin I zu erwarten war. Weiter unterscheidet sich die Serumkonzentrationskurve von Troponin T, beispielsweise beim transmuralen Infarkt, signifikant von der Kurve des Troponin I. Es wird im Gegensatz zu Troponin I anstelle einer zweiphasigen eine dreiphasige Verlaufskurve gefunden, mit der Troponin T im Mittel als erhöht bis zu 300 Stunden nach Schmerzbeginn gefunden wird. Das Zeitintervall absoluter diagnostischer Sensitivität erstreckt sich von der 6. bis zur 195. Stunde. Damit ist das Zeitintervall absoluter diagnostischer Sensitivität nahezu viermal so lang wie es für Troponin I bekannt ist. Weiter wurde gefunden, daß durch die Troponin T-Bestimmmung die Verlaufskontrolle einer thrombolytischen Therapie möglich ist.

Für die erfindungsgemäß immunologische Bestimmungsmethode eignen sich im Prinzip alle gängigen Immunoassays, wie Radioimmunoassay, Enzymimmunoassay, Fluoreszenzimmunoassay usw.. Ferner sind sämtliche Verfahrensvarianten, wie kompetitiver Immunoassay, IEMA-Verfahren usw., anwendbar. Zur Bestimmung von Troponin T hat sich als besonders zweckmäßig ein Sandwich-Test erwiesen. Bei diesem Testverfahren wird ein immobilisierter Antikörper gegen Troponin T und ein Konjugat aus einem Antikörper gegen Troponin T und einer bestimmbaren Gruppe verwendet. Die verschiedenen Varianten dieser Testmethode sowie Einzelheiten zur Durchführung dieser Verfahren sind in der Literatur ausführlich beschrieben. Es sind jedoch auch andere immunologische Bestimmungsverfahren unter Verwendung der erfindungsgemäßen Antikörper möglich, so wie sie z. B. in der deutschen Patentanmeldung DE-A 38 34 766 und/oder-DE-A 38 22 750 beschrieben sind, möglich.

Unter einem Antikörper im Sinne der Erfindung ist ein kompletter Antikörper, chimärer Antikörper, bivalenter Antikörper oder Fragmente davon zu verstehen. Die verwendeten Antikörper gegen Troponin T können polyklonal oder monoklonal sein. Die Verwendung von monoklonalen Antikörpern ist bevorzugt.

Beim Sandwich-Test als bevorzugte Ausführungsform wird die Probenlösung mit mindestens zwei Antikörpern gegen Troponin T inkubiert. Der erste Antikörper vermittelt dabei die Bindung an die Festphase. Dazu kann dieser Antikörper entweder direkt oder über einen Spacer an die Festphase gebunden sein oder aber in löslicher Form vorliegen und erst nach Durchführung der immunologischen Reaktion immobilisiert werden. Der zweite Antikörper kann entweder direkt mit einer bestimmten Gruppe markiert sein oder über eine funktionelle Bindung an die bestimmbare Gruppe gebunden werden. Dazu kann der Antikörper an den einen Partner eines spezifischen Bindungspaars und die bestimmbare Gruppe an den an den anderen Partner des spezifischen Bindungspaars gebunden sein. Bei der Umsetzung entsteht dann ein Komplex, der sowohl den zweiten Antikörper als auch die bestimmbare Gruppe enthält. Die Bindung des ersten Antikörpers an den Träger (Immobilisierung) erfolgt nach den dem Fachmann geläufigen Methoden durch adsorptive, chemische Bindung oder durch funktionelle Bindung über ein spezifisches Bindungspaar. In diesem Falle ist ein Partner des Bindungspaares immobilisiert, während der andere Partner chemisch an den Antikörper gebunden ist. Über dieses Bindungspaar kann dann der Antikörper vor oder während der immunologischen Bestimmungsreaktion immobilisiert werden. Beispiele für derartige Bindungspaare sind Biotin-Streptavidin/Avidin, Hapten-Antikörper, Antigen-Antikörper, Zucker-Lectin, Hapten-Bindeprotein.

Eine weitere bevorzugte Testvariante ist der kompetitive Test. Dabei wird ein vor oder während der Bestimmung immobiliserter Antikörper gegen Troponin T und ein Konjugat aus Troponin T und einer bestimmbaren Gruppe verwendet.

Als Trägermaterialien zur Immobilisierung der erfindungsgemäßen Antikörper bzw. zur Immobilisierung von Troponin T können Materialien verwendet werden, wie z. B. Röhrchen (tubes), Kunststoffküvetten, Mikrotiterplatten, Kugeln (beads) oder Mikrocarrier aus Kunststoffen, wie Polystyrol, Vinylpolymere, Polypropylen, Polycarbonat, Polysaccharide, Silikone, Gummi oder behandeltes Glas (vgl. z. B. E.T. Maggio "Enzyme Immunoassay" CAC. Press, Florida (1980), insbesondere Seiten 175 - 178, EP-A-O63064, Bioengineering 16 (1974), 997 - 1003, C.J. Senderson und D.V. Wilson, Immunology 20 (1971), 1061 - 1065). Insbesondere wird als Trägermaterial ein mit Avidin oder Streptavidin beschichtetes Trägermaterial, insbesondere Polystyrol, vorzugsweise hergestellt wie in EP-A-0269092, beschrieben. Der Bindepartner B enthält ebenfalls entweder Troponin T oder ein Analogon davon oder einen mit spezifisch mit Troponin T bindefähigen monoklonalen Antikörper und ist markiert. Zur Markierung eignen sich die für die jeweilige Bestimmungsmethode üblichen Mittel. So werden bei einem Radioimmunoassay Radioisotope, beispielsweise $^{57}$Co zur Markierung verwendet. Für einen Enzymimmunoassay sind sämtliche hierfür üblicherweise eingesetzte Enzyme, beispielsweise Peroxidase oder β-Galactosidase geeignet. Für einen Fluoreszenz-Immunoassay sind die üblichen fluoreszierenden Gruppen als Marker brauchbar. Einzelheiten dieser verschiedenen Testmethoden und Verfahrensvarianten sind dem Fachmann bekannt. In analoger Weise wie bei der Bindung an die Festphase kann auch die Bindung der Markierung an Troponin T bzw. den Antikörper kovalent oder über ein spezifisches Bindungspaar erfolgen.

Die kovalente Bindung des Antikörpers bzw. von Troponin T an einen der o.g. Bindungspartner erfolgt nach den dem Fachmann geläufigen Methoden, beispielsweise über Carbodiimid und Hydroxysuccinimid.

Als Markierungsenzym wird vorzugsweise Peroxidase (POD) verwendet.

Die aus dem Stand der Technik bekannten monoklonalen Antikörper gegen Herzmuskel-Troponin T sind Spezies-unspezifisch und zeigen starke Kreuzreaktivitäten gegenüber Skelettmuskel-Troponin T (Eur. J. Biochem. 150, (1985), 517-525; J. Biochem. (Tokyo) 102 (1987), 25-30).

Ein weiterer Gegenstand der Erfindung ist daher ein Verfahren zur Herstellung von Herzmuskel-Troponin T spezifisch bindefähigen polyklonalen Antikörpern, deren Kreuzreaktion mit humanem Skelettmuskel-Troponin T weniger als 5 % und mit Troponin I und anderen myofibrillären Proteinen weniger als 2 % beträgt. Das Verfahren besteht darin, daß man Versuchstiere, vorzugsweise Schafe, mit humanem Herzmuskel-Troponin T, in Kombination mit einem Adjuvans, über mehrere Monate (vorzugswweise 4-6 Monate) mit mindestens vier Immunisierungen in vier- bis sechswöchigem Abstand immunisiert, Rohserum gewinnt und dieses immunsorptiv reinigt.

Ein weiterer Gegenstand der Erfindung ist ein Verfahren zur Herstellung von mit Herzmuskel-Troponin T spezifisch bindefähigen monoklonalen Antikörpern deren Kreuzreaktion mit humanem Skelettmuskel-Troponin T weniger als 5% und mit Troponin I und anderen myofibrillären Proteinen weniger als 2% beträgt. Das Verfahren besteht darin, daß man Versuchstiere, vorzugsweise Inzuchtmäuse, mit humanem Herzmuskel-Troponin T, in Kombination mit einem Adjuvans über mehrere Monate mit mindestens vier Immunisierungen in vier- bis sechswöchigem Abstand intraperitoneal immunisiert, B-Lymphozyten gewinnt, mit einer permanenten Myelomzellinie fusioniert, die Klone isoliert und hieraus die Antikörper gewinnt.

Bevorzugt wird als Adjuvans Aluminiumhydroxid zusammen mit Bordatella pertussis oder Freund'schem Adjuvans eingesetzt.

Die bei der Fusionierung nach dem bekannten Verfahren von Köhler und Milstein (Nature 256 (1975), 495 -497) gewonnenen Primärkulturen von Hybridzellen werden in üblicher Weise, z. B. unter Verwendung eines handelsüblichen Zellsorters oder durch "Limiting Dilution" kloniert. Es werden jeweils die Kulturen weiterverarbeitet, die positiv gegenüber isoliertem Herzmuskel-Troponin T und gegenüber Herzmuskel-Troponin T im Patientenserum reagieren und negativ mit Troponin T aus Skelettmuskel reagieren. Man erhält auf diese Weise Hybridoma-Zellinien, die die erfindungsgemäßen monoklonalen Antikörper produzieren. Nach bekannten Methoden können diese Zellinien kultiviert und die von ihnen produzierten monoklonalen Antikörper isoliert werden.

Beispiele für die auf diese Weise gewonnenen Hybridoma-Zellinien sind Klon 7.1 A 12.2-22 (ECACC 89060901) und Klon 8.1F 6.7-2 (ECACC 89030308). Die Zellinien sind unter der jeweils angegebenen Nummer bei der European Collection of Animal Cellcultures, GB, hinterlegt.

Die so gewonnenen polyklonalen oder monoklonalen Antikörper zeichnen sich durch eine geringe Kreuzreaktivität gegenüber humanem Skelettmuskel-Troponin T aus, die unter 5 %, vorzugsweise unter 2 % liegt und durch eine Kreuzreaktivität gegenüber Troponin I und gegenüber anderen myofibrillaren Proteinen von unter 2 %.

Die erfindungsgemäßen polyklonalen und monoklonalen Antikörper eigenen sich hervorragend dazu, in einer Probe, beispielsweise Serum oder Plasma, Herzmuskelnekrosen spezifisch zu bestimmen. Für diese Bestimmungsverfahren können die Antikörper als solche, als chimäre Antikörper oder Fragmente hiervon, welche die entsprechenden immunologischen Eigenschaften aufweisen, beispielsweise als Fab-Fragmente, verwendet werden. Unter dem Begriff Antikörper werden daher sowohl vollständige Antikörper als auch Fragmente davon verstanden.

Die nachfolgenden Beispiele und Abbildungen sollen die Erfindung näher erläutern, ohne sie darauf zu beschränken. Prozentangaben beziehen sich auf Gew.-%.

Fig. 1    zeigt eine Standardkurve für eine Troponin T Bestimmung

Fig. 2    zeigt die mittlere Serumkonzentration von Toponin T für 52 Patienten mit transmuralem Myokardinfarkt.

Fig. 3    zeigt die mittlere Serumkonzentration von Troponin T für 20 Patienten mit Reperfusion des Infarktareals früher als 3,5 Stunden nach Schmerzbeginn (ER), für 20 Patienten mit Reperfusion später als 3,5 Stunden (LR) nach Schmerzbeginn und für 24 Patienten mit nicht-reperfundiertem Infarkt (PO).

Beispiel 1

Isolierung von Troponin T

Puffer 1:
    0,05 mol/l Tris/HCl, pH 7,0

0,05 mol/l KCl

10 ml/l Triton™ X 100

2 mmol/l EDTA

0,5 mmol/l DTT (Dithiothreit)

0,02 % Natriumazid

2 mmol/l PMSF (Phenylmethylsulfonylfluorid)

5 mmol/l ξ-Aminocapronsäure

2,5 ml Trasylol/l

0,2 mg Pepstatin/l

Puffer 2:

0,05 mol/l Tris/HCl, pH 7,0

1 mol/l KCl

5 mol/l Harnstoff

2 mmol/l EDTA

0,02 % Natriumazid (w/v)

Puffer 3:

5 mmol/l Kaliumphosphat, pH 7,3

2 mol/l Harnstoff

0,6 mol/l KCl

0,5 mmol/l DTT

0,02 % Natriumazid (w/v)

Puffer 4:

0,05 mol/l Tris/HCl, pH 7,8

6 mol/l Harnstoff

0,01 mol/l NaCl

2 mmol/l EDTA

0,5 mmol/l DTT

0,02 % Natriumazid (w/v)

300 g Gewebe (Linker Ventrikel ohne Klappen und Gefäße) wird kleingeschnitten und in dem fünffachen Volumen Puffer 1 bei 4°C im Mixer maximal 1 Minute in Intervallen homogenisiert. Der nichtlösliche Anteil wird bei 27500 g und 4°C über 15 min abzentrifugiert und das Pellet solange mit 1 l Puffer 1 gewaschen bis der Überstand völlig klar ist.

Das Pellet wird in 5 - 6fachem Volumen Puffer 2 aufgenommen und 3 - 4 Stunden bei 4°C gerührt. Es wird bei 27500 g und 4°C 15 min zentrifugiert.

Der Überstand wird mit 5 mmol/l ATP versetzt (Endkonzentration nach Ammonsulfatzugabe) und durch Zugabe von bei 4°C gesättigter Ammonsulfatlösung/2 mmol/l EDTA auf 35 % Ammonsulfat gebracht, wobei die Ammonsulfatlösung vorher mit KOH neutralisiert wird. Es wird über Nacht bei 4°C und leichtem Rühren inkubiert. Anschließend wird der Niederschlag bei 27500 g und 4°C 20 min abzentrifugiert.

Der Überstand wird durch Zugabe von festem Ammonsulfat auf 45 % Ammonsulfat-Sättigung gebracht, 45 min bei 4°C gerührt und abzentrifugiert. Die 35 - 45 % Ammonsulfatfällung enthält Troponin T und wird weiterverarbeitet. Das Pellet wird in 25 ml Puffer 3 aufgenommen (OD 280 nm soll zwischen 1,5 und 2 liegen) und gegen Puffer 3 bei 4°C bei mindestens zweimaligem Pufferwechsel dialysiert.

Die dialysierte Lösung wird auf eine Hydroxylapatitsäule (Biogel HTP, Beladung ca. 25 mg Protein/50 ml Säulenvolumen, Fluß 1 Säulenvolumen/Stunde) aufgetragen, mit 4 - 5 Säulenvolumen Puffer 3 nachgewaschen und mit einem Gradienten von 5 mmol/l bis 55 mmol/l Kaliumphosphat in Puffer 3 innerhalb von 20 Stunden bei einem Säulenvolumen/Stunde eluiert. Die Fraktionen, die Troponin-T enthalten, werden mittels SDS-Page (Gradientengel 5 -25 %) ermittelt, gepoolt und gegen Puffer 4 dialysiert.

Die dialysierte Lösung wird auf eine Säule (DEAE-Servacel SS23, Säulenvolumen 20 - 30 ml, Fluß 1 Säulenvolumen/Stunde) aufgegeben, mit 3 Säulenvolumen Puffer 4 nachgewaschen und mit einem Gradienten von 0,01 mol/l bis 0,25 mol/l NaCl in Puffer 4, innerhalb 20 Stunden bei 1 Säulenvolumen/Stunde eluiert. Troponin I erscheint im Durchlauf. Troponin T und Troponin C eluieren getrennt. Die Troponin-T-haltigen Fraktionen werden nach SDS-page gepoolt, gegen Puffer 4 umdialysiert und zur weiteren Verwendung bei -20°C tiefge-froren. Aus 300 g Muskel werden ca. 6 - 8 mg Troponin T erhalten.

Beispiel 2

Gewinnung von monoklonalen Antikörpern gegen humanes Troponin-T

Balb/c-Mäuse, 8-12 Wochen alt, werden mit 100 μg Troponin T (isoliert aus humanem Herzmuskel nach Beispiel 1), mit komplettem Freund-schen Adjuvans, intraperitoneal immunisiert. Nach 6 Wochen werden drei weitere Immunisierungen in 4 wöchigem Abstand durchgeführt. Dabei werden jeweils 50 μg Troponin T, an Aluminiumhydroxid und Bordetella pertussis adsorbiert, intraperitoneal verabreicht.

Eine Woche nach der letzten Immunisierung erfolgt eine Blutabnahme und die Bestimmung des Antikörpertiter im Serum der Versuchstiere.

Bei positivem Verlauf der Immunisierung wird fusioniert. Vier Tage, drei Tage und zwei Tage vor der Fusionierung wird jeweils noch einmal mit 100 μg Troponin T in PBS (=phosphat buffered saline) intravenös immunisiert.

Zur Fusion werden in Anlehnung an Galfre, (Methods in Enzymology, 73, 1981, S. 3) 1 x 10⁸ Milzzellen einer immunisierten Maus mit 2 x 10⁷ Myelomzellen (P3x63Ag8-653, ATCCCRL 8375) gemischt und anschließend 10 Minuten zentrifugiert (300 g, 4° C). Die Zellen werden mit serumfreiem Kulturmedium gewaschen und bei 400 g erneut zentrifugiert. Der Überstand wird abgesaugt und das Zellsediment wird mit 1 ml 50 %iger PEG-Lösung (MG 4000, Fa. Merck) versetzt. Danach wird bei Raumtemperatur mit RPMI 1640-Medium (RPMI= Rosewell Parker Memory Institut) ohne fötalem Kälberserum (FKS), langsam ausverdünnt ( 20 ml in 15 Minuten). Die Zellsuspension wird anschließend 10 Minuten bei 400 g zentrifugiert und das Zellsediment in Selektionsmedium aufgenommen (RPMI 1640, 10% FKS, Hypoxanthin 100 mmol/l, Azaserin 1 μg/ml). An Wachstumsfaktoren wird HECS (Human Endothelial Culture Supernatant) als Feederzellersatz verwendet (Fa. Costar, No. 6110).

Die fusionierten Zellen werden 5 x 10⁴ Zellen pro Vertiefung in 24-well Platten (Fa. Nunc) ausplattiert. Nach 7-10 Tagen ist Klonwachstum sichtbar. Der Kulturüberstand der Primärkulturen wird nach einem in Beispiel 3 beschriebenen ELISA-Verfahren getestet. Die Primärkulturen, die Antigen-spezifische Antikörper enthalten, werden mit Hilfe eines fluoreszenzaktivierenden Zellsorters auf 96-well Zellkulturplatten (Fa.Nunc) weiter kloniert. Als Feederzellersatz wird HECS (siehe oben) eingesetzt.

Auf diese Weise konnten die beiden Hybridoma-Zellinien Klon 7.1A12.2-22 und 8.1F6.7-2 isoliert werden, die bei der Hinterlegungsstelle ECACC (European Collection of Animal Cell Cultures) unter den Hinterlegungsnummern

ECACC 89060901 (= Klon 7.1A12.2-22) und
ECACC 89030308 (= Klon 8.1F6.7-2)
hinterlegt worden sind.

Zur Erzeugung von Aszites werden 5 x 10⁶ Hybridomzellen intraperitoneal in Balb/c Mäuse gespritzt, die zuvor 1-2 mal mit 0,5 ml Pristan vorbehandelt worden sind. Nach 2-3 Wochen kann aus dem Bauchraum der Mäuse Ascites-Flüssigkeit gewonnen werden. Hieraus können in üblicher Weise die Antikörper isoliert werden. Diese monoklonalen Antikörper sind spezifisch gegen humanes Herzmuskel-Troponin T gerichtet und zeigen keine bzw. nur geringe Kreuzreaktivität mit Skelettmuskel-Troponin T. Sie werden im folgenden mit MAK 7.1A12.2-22 (von Klon 7.1A12.2-22) bzw. MAK 8.1F6.7-2 (von Klon 8.1F6.7-2) bezeichnet.

Die beiden monoklonalen Antikorper gehören der Subklasse IgG1/kappa an.

Beispiel 3: Screening-Test auf Antikörper gegen humanes Herzmuskel-Troponin-T

Um die Anwesenheit und Spezifität von Antikörpern gegen Troponin T im Serum immunisierter Mäuse, im Kulturüberstand der Hybridzellen, oder in Aszitesflüssigkeit zu erkennen, wird ein ELISA-Verfahren als Testprinzip verwendet: Mikrotiterplatten werden mit 10 μg/ml polyklonalem Antikörper (IgG) gegen humanes Troponin T aus Schaf (immunsorptiv gereinigt, keine Kreuzreaktivität mit Skelettmuskel-Troponin T, Kreuzreaktion mit Herzmuskel-Troponin T, Herstellung nach Beispiel 7) in Beschichtungspuffer (0,2 mol/l Natriumcarbonat/-Bicarbonat, pH 9,3 -9,5) 1 Stunde bei Raumtemperatur beschichtet. Die Nachbeschichtung erfolgt mit 0,9% Natriumchloridlösung und 1% Rinderserumalbumin für 20 Minuten. Anschließend wird mit Waschpuffer gewaschen ( 0,9% Natriumchlorid-lösung). Die Inkubation des Antigens, humanes gereinigtes Troponin T 1 μg/ml bzw. "natives" Troponin T d.h. Infarktserum ( 1:2 verdünnt), erfolgt mit 100 μl pro well bei Raumtemperatur, 1 Stunde unter Schütteln. Dann wird erneut zweimal mit Waschpuffer gewaschen. Die Probeninkubation erfolgt mit 100 μl pro well, 1 Stunde, bei Raumtemperatur, unter Schütteln. Dann wird erneut zweimal mit Waschlösung gewaschen. Dann erfolgt eine weitere Inkubation mit 25 mU eines PAK<M-Fcg>S-Fab(IS)-Peroxidasekonjugates, 100 μl pro well, für 1 Stunde bei Raumtemperatur unter Schütteln. (PAK<M-Fcg>S-Fab(IS) = Fab-Fragment eines polyklonalen anti-Maus Fc-γ-Antikörpers aus Schaf, der immunsorptiv gereinigt ist.) Nach ei-

6

nem Waschschritt mit Waschpuffer wird die Peroxidaseaktivität in üblicher Weise bestimmt (beispielsweise mit ABTS 30 Minuten bei Raumtemperatur, abgelesen wird die Extinktionsdifferenz in mE, bei 405 nm).

Beispiel 4

Bestimmung der Kreuzreaktivität mit humanem Skelettmuskel-Troponin T

Es wird, wie in Beispiel 3 beschrieben, vorgegangen. Zunächst wird die Reaktivität von Herzmuskel-Troponin T bestimmt. Dann wird zu dem jeweiligen monoklonalen Antikörper das auf Kreuzreaktion zu prüfende Antigen (Skelettmuskel-Troponin T) in steigenden Konzentrationen zugegeben.

Die Kreuzreaktionen werden anschließend nach folgender Formel berechnet:

$$\frac{C \ (Herzmuskel-Troponin \ T)}{C \ (Skelettmuskel-Troponin \ T)} \times 100 \ = \ \% \ Kreuzreaktion$$

C = Konzentration des Antigens, die zur Erreichung von 50% des maximalen Signals erforderlich ist.

Beispiel 5:

Bestimmung der Epitopspezifität

Eine Mikrotiterplatte wird mit 10 µg/ml polyklonalem Schaf-Antikörper gegen die Fcg-Region eines Maus-Antikörpers in 0,2 mmol/l Carbonatpuffer, pH 9,6, 1 Std. bei Raumtemperatur oder über Nacht bei 4°C beschichtet. Danach wird mit Inkubationspuffer (0,9 % Natriumchloridlösung und 1% Rinderserumalbumin) 20 Minuten nachbeschichtet und dann mit Waschpuffer gewaschen (0,9% Natriumchloridlösung, 0,05% Tween™ 20). Anschließend werden 100 µl eines monoklonalen Antikörpers (MAK 7.1A12.2-22, MAK 1) 10 µg/ml in Inkubationspuffer (0,9% Natriumchloridlösung mit 1% Rinderserumalbumin) zugegeben und 1 Stunde bei Raumtemperatur, unter Schütteln inkubiert.

Ein zweiter monoklonaler Antikörper (MAK 8.1F6.7-2, MAK 2), der als Peroxidase Konjugat vorliegt, wird in Lösung (100 mU/ml) bei Raumtemperatur mit dem Antigen (Herzmuskel-Troponin-T) 1 µg/ml, eine Stunde vorinkubiert.

Nach der Inkubation der Platte mit MAK 1 wird überschüssiger Antikörper durch Waschen entfernt. Die Platte wird anschließend mit 1% Maus-Normalserum in Inkubationspuffer nachbeschichtet. 100 µl des vorinkubierten Troponin T/MAK 2-Peroxidase-Komplexes wird auf die Platte gegeben und 1 Stunde, bei Raumtemperatur unter Schütteln inkubiert. Die gebundene Peroxidase-Aktivität wird mit ABTS als Substrat sichtbar gemacht. Erkennt der MAK 2 dasgleiche bzw. ein überlappendes Epitop wie MAK 1, so kommt es zu keiner Paarungsbildung zwischen MAK 1/Troponin T/MAK 2 und somit zu keiner Substratreaktion. Die gefundenen Ergebnisse zeigen, daß die beiden monoklonalen Antikörper 7.1A12.2-22 und 8.1F6.7-2 gegen verschiedene Epitope des Antigens Herzmuskel-Troponin-T gerichtet sind.

Beispiel 6

Enzym-Immunoassay zur Bestimmung von Troponin T nach dem ELISA-Prinzip

Reagenz 1
1,25 µg/ml biotinylierter MAK 7.1A12.2-22 (Herstellung analog J. H. Peters et al., Monoklonale Antikörper, Springer Verlag, Berlin, 1985, Seiten 209 - 212.)
10 mmol/l Citratpuffer
47 mmol/l Phosphatpuffer, pH 6,3
50 mU/ml Konjugat aus Peroxidase und monoklonalem Antikörper 8.1F6.7-2 (hergestellt analog M. B. Wilson, P. K. Nakane (1978) in Immunofluorescence and Related Staining Techniques (W. Knapp, K. Kolubar, G. Wick eds.) pp. 215 - 224, Elsevier/North Holland, Amsterdam, die Aktivitätsangabe bezieht sich auf Peroxidase).
Reagenz 2
100 mmol/l Phosphat-Citratpuffer, pH 4,4
3,2 mmol/l Natriumperborat
1,9 mmol/l ABTS (2,2'-Azino-di-[3-ethyl-benzthiazolinsulfonat(6)]
Als Probe werden Humanseren verwendet, die mit 3, 5, 10 bzw. 25 ng/ml Troponin T aufgestockt sind.

Die Reaktion wird durchgeführt mit streptavidinbeschichteten Polystyrolröhrchen (Herstellung nach EP-A-0269092).

Durchführung der Bestimmung:

0,2 ml Probe werden mit 1 ml Reagenz 1 60 min bei 20 - 25°C in einem Röhrchen inkubiert. Es wird ausgesaugt und zweimal mit Leitungswasser gewaschen. Anschließend wird Reagenz 2 zugegeben, 30 min bei 20 - 25°C inkubiert und bei 420 nm im Photometer die Extinktion bestimmt.

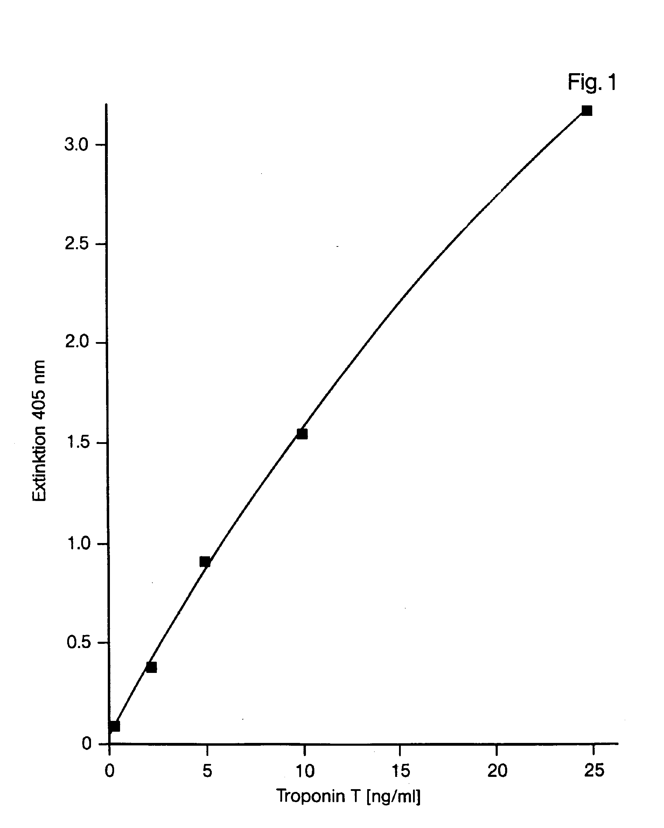

Auf diese Weise wird eine Standardkurve (Abb. 1) erhalten, über die die Troponin T-Konzentrationen von Patientenproben bestimmt werden können.

Beispiel 7

Gewinnung und Reinigung von polyklonalen Antikörpern gegen Troponin T

Schafe werden mit 55µl einer Lösung von 0,1 mg/ml humanem Troponin T in komplettem Freundschen Adjuvans (CFA) erstimmunisiert. Weitere Immunisierungen erfolgen am 7., 14. und 30. Tag. Im weiteren wurde alle 30 Tage immunisiert. Bei diesen Folgeinjektionen wurden jeweils 28 µl einer Troponin T-Lösung von 0,05 mg/ml in CFA verwendet. Nach sechs Monaten wird das Rohserum gewonnen.

1 l Rohserum wird mit 15 g Aerosil™ (Hersteller: Degussa) versetzt, 1 Stunde bei Raumtemperatur gerührt und zentrifugiert. Anschließend wird der Überstand mit 1,7 mol/l Ammonsulfat versetzt und 2 Stunden bei Raumtemperatur langsam gerührt. Anschließend wird der Niederschlag abzentrifugiert und in 0,2 l Dialysepuffer (15 mmol/l Kaliumphosphat, 50 mmol/l Natriumchlorid, pH 7,0) homogenisiert und bei 4°C gegen 4 x 10 l Dialysepuffer dialysiert. Nach erneuter Zentrifugation wird das dialysierte Produkt über DE 52-Cellulose, unter Elution mit Dialysepuffer, gereinigt.

Das Eluat wird ad 50 mmol/l Kaliumphosphat, pH 7,5, 150 mmol/l Natriumchlorid (PBS), 0,1 % Azid aufgestockt und mit einer Konzentration von ca. 15 mg/ml Protein bei Raumtemperatur über eine Troponin T-Immunadsorbens-Säule gegeben und mit PBS/0,1 % Azid proteinfrei gewaschen. Anschließend wird mit 1 mol/l Propionsäure eluiert.

Die Herstellung des Troponin T-Immunadsorbers erfolgt dadurch, daß mit 15 %iger Salpetersäure und $H_2O$ gewaschenes Spherosil™ (Rhone Poulenc XOC005) nach Trocknen mit 10 % (v/v) 3-(Triethoxysilyl)propylamin in DMSO bei 85°C über Nacht zu Spherosil-$NH_2$ umgesetzt wird. Nach der Umsetzung wird mit DMSO und Isopropanol gewaschen und das Adsorbens bei 50°C getrocknet.

Spherosil-$NH_2$ wird mit 10 %iger Glutardialdehyd-Lösung, pH 3,7, vermischt und bei 55°C 2 Stunden erwärmt. Die Suspension wird anschließend unter Vakuum über einen Glasfilter abgesaugt. Dann wird mit dem siebenfachen Spherosilvolumen an destilliertem Wasser gewaschen und mit dem fünffachen Volumen an 10 mmol/l Kaliumphosphat, pH 8,0/0,1 mol/l Natriumchlorid nachgewaschen. 10 mg Troponin T werden pro ml Spherosil in 10 mmol/l Kaliumphosphat, pH 8/0,1 mol/l Natriumchlorid in ca. 50 % des eingesetzten Spherosil-Volumens in einem Rundkolben zugegeben. Der Kolben wird über Nacht bei Raumtemperatur am Rotationsverdampfer rotiert.

Nach Filtration wird das Spherosil mehrfach mit 0,9 %iger NaCl-Lösung sowie Äthanolamin-Lösung nachgewaschen. Anschließend werden 3 Volumenteile Äthanolamin-Lösung zugegeben und 1 Stunde bei Raumtemperatur inkubiert. Nach nochmaliger Filtration wird wieder mit NaCl-Lösung gewaschen. Anschließend wird der Immunadsorber mit PBS auf pH 7,5 eingestellt und mit PBS/Natriumazid äquilibriert. Die Lagerung erfolgt bei 4°C.

Beispiel 8

Bestimmung von Herzmuskelnekrosen

Es wurde mit Serum von 37 Patienten mit instabiler Angina pectoris-Symptomatik ein Enzym-Immunoassay zur Bestimmung von Troponin T nach Beispiel 6 durchgeführt.

Bei 13 dieser Patienten (30 %) konnte eine signifikante Erhöhung von Troponin T gefunden werden. Dies spricht für die deutlich höhere Sensibilität der Troponin T-Bestimmung zum Nachweis des kleinen Infarkts, verglichen mit dem Troponin I-Test und der Bestimmung erhöhter Aktivitäten kardialer Enzyme.

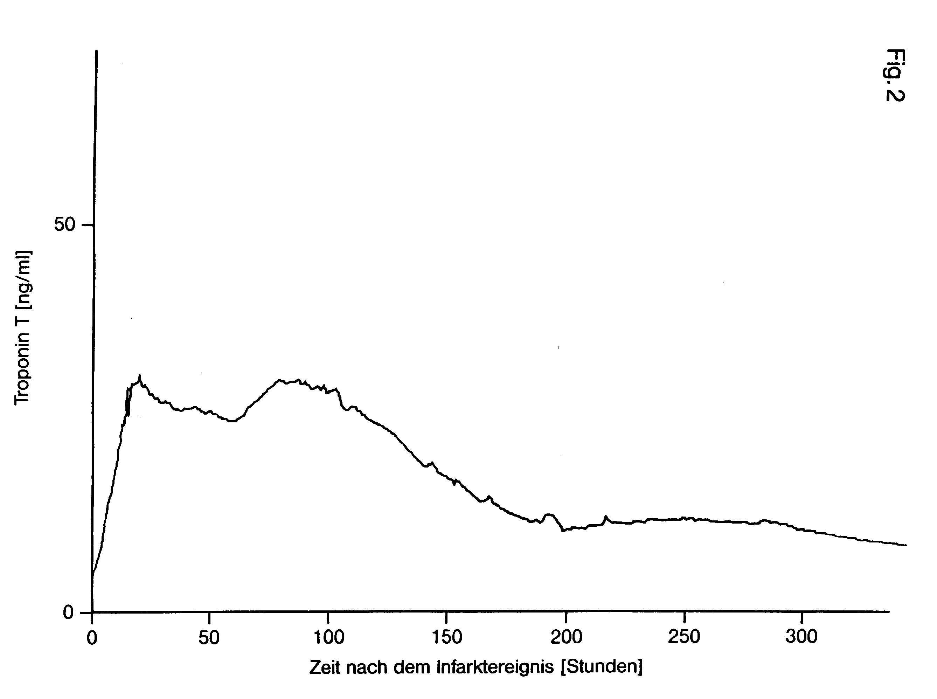

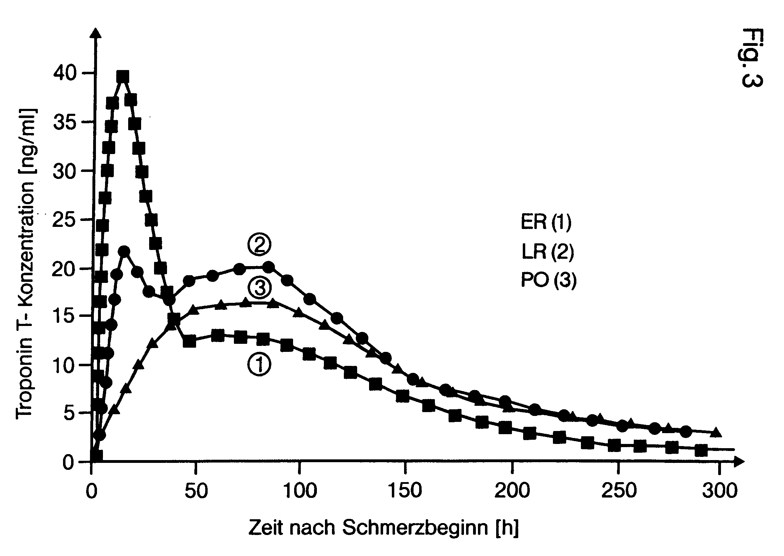

Auch beim transmuralen Infarkt unterscheidet sich die Serumkonzentrationskurve von Troponin T signifikant von der von Troponin I. Dies wird durch Abbildung 2 belegt, welche die mittlere Serumkonzentration von Troponin T für 52 Patienten mit einem transmuralen Myokardinfarkt zeigt. So wird Troponin T in dieser Infarktgruppe (transmuraler Infarkt) im Mittel länger als 300 Stunden nach Schmerzbeginn erhöht gefunden (Abb. 3). Das Zeitintervall absoluter diagnostischer Sensitivität erstreckt sich von der 6. bis zur 195. Stunde. In einer größeren Gruppe von Patienten mit sehr unterschiedlichen Infarkten (Q-wave and non-Q-wave AMI) erstreckt sich das Zeitintervall absoluter diagnostischen Sensitivität von Troponin T von 12 bis 140 Stunden nach Schmerzbeginn.

Die Troponin T Freisetzung zeigt einen Verlauf, der sowohl für cytosolische und strukturell gebundene Markerproteine charakteristisch ist. Im reperfundierten AMI wird ein deutlicher Troponin T Peak am Tag 1 gefunden, während dieser Peak bei nicht-reperfundiertem AMI nicht auftritt (Abb. 3). Mit der perfusionsabhängigen Änderung der Toponin T Freisetzung in der Frühphase des Myokardinfarkts kann nicht invasiv die Effektivität einer thrombolytischen Therapie vorausgesagt werden. Die ist mit anderen Markerproteinen wie beispielsweise Troponin I nicht möglich.

## Patentansprüche

1. Verfahren zur Bestimmung von Herzmuskelnekrosen nach dem Prinzip des Immunoassays, dadurch gekennzeichnet, daß eine Serum- oder Plasmaprobe mit mindestens einem Antikörper gegen Troponin T und einem Bindepartner B für Troponin T oder für den Antikörper inkubiert wird, wobei entweder der Antikörper gegen Troponin T oder der Bindepartner B mit einer bestimmbaren Gruppe markiert ist, der dabei gebildete immunologische Komplex, der die bestimmbare Gruppe enthält, abgetrennt und die bestimmbare Gruppe in der abgetrennten oder noch verbliebenen Phase als Maß für Troponin T aus der Probe bestimmt wird.

2. Verfahren nach Anspruch 1, dadurch gekennzeichnet, daß die Probe mit einem Antikörper gegen Troponin T und einem Konjugat aus einem Antikörper gegen Troponin T und einer bestimmbaren Gruppe inkubiert wird, der gebildete immunologische Komplex durch Phasentrennung abgetrennt und die bestimmbare Gruppe in einer der Phasen bestimmt wird.

3. Verfahren nach Anspruch 1, dadurch gekennzeichnet, daß die Probe mit einem Antikörper gegen Troponin T und einem Konjugat aus Troponin T und einer bestimmbaren Gruppe inkubiert wird, der gebildete immunologische Komplex durch Phasentrennung abgetrennt und die bestimmbare Gruppe in einer der Phasen bestimmt wird.

4. Verwendung von Antikörpern gegen Troponin T zur Bestimmung von Herzmuskelnekrosen.

5. Monoklonale oder polyklonale Antikörper gegen Troponin T mit einer Kreuzreaktivität von <2 % gegen Troponin I und andere myofibrillare Proteine sowie von <5% gegenüber Skelettmuskel-Troponin T.

6. Hybridoma-Zellinien ECACC 89060901 und ECACC 89030308

7. Monoklonale Antikörper hergestellt unter Verwendung der Hybridoma-Zellinien ECACC 89060901 und ECACC 89030308.

8. Verfahren zur Herstellung von polyklonalen Antikörpern gegen Troponin T mit einer Kreuzreaktivität von <2 % gegen Troponin I und andere myofibrillare Proteine, sowie von <5 % gegenüber Skelettmuskel-Troponin T durch Immunisierung von Versuchstieren mit humanem Herzmuskel-Troponin T in Kombination mit einem Adjuvans über mehrere Monate mit mindestens 4 Immunisierungen in vier- bis sechswöchigem Abstand immunisiert, Rohserum gewinnt und dieses immunsorptiv reinigt.

9. Verfahren zur Herstellung von monoklonalen Antikörpern gegen Troponin T mit einer Kreuzreaktivität von <2 % gegen Troponin I und andere myofibrillare Proteine, sowie von <5 % gegenüber Skelettmuskel-Troponin T durch Immunisierung von Versuchstieren mit humanem Herzmuskel-Troponin T in Kombination mit einem Adjuvans über mehrere Monate mit mindestens 4 Immunisierungen in vier- bis sechswöchigem Abstand immunisiert, B-Lymphozyten gewinnt, diese mit einer permanenten Myelomzellinie fusioniert, die Klone isoliert und hieraus die Antikörper gewinnt.

## Claims

1. Process for the determination of heart muscle necroses according to the principle of immunoassay, characterised in that a serum or plasma sample with at least one antibody against troponin T and a binding partner B for troponin T or for the antibody is incubated, whereby either the antibody against troponin T or the binding partner B is labelled with a determinable group, the thereby formed immunological complex

EP 0 394 819 B1

which contains the determinable group is separated off and the determinable group in the separated off or still remaining phase is determined as measure for troponin from the sample.

2. Process according to claim 1, characterised in that the sample with an antibody against troponin T and a conjugate from an antibody against troponin T and a determinable group is incubated, the formed immunological complex is separated by phase separation and the determinable group is determined in one of the phases.

3. Process according to claim 1, characterised in that the sample with an antibody against troponin T and a conjugate of troponin T and a determinable group is incubated, the formed immunological complex is separated off by phase separation and the determinable group is determined in one of the phases.

4. Use of antibodies against troponin T for the determination of heart muscle necroses.

5. Monoclonal or polyclonal antibody against troponin T with a cross-reactivity of < 2% against troponin I and other myofibrillary proteins, as well as of < 5% towards skeletal muscle troponin T.

6. Hybridoma cell lines ECACC 89060901 and ECACC 89030308.

7. Monoclonal antibodies produced with use of the hybridoma cell lines ECACC 89060901 and ECACC 89030308.

8. Process for the production of polyclonal antibodies against troponin T with a cross-reactivity of < 2% against troponin I and other myofibrillary proteins, as well as of < 5% towards skeletal muscle troponin T, by immunisation of experimental animals with human heart muscle troponin T in combination with an adjuvant for several months with at least 4 immunisations in four to six week intervals, crude serum is obtained and this is immunosorptively purified.

9. Process for the production of monoclonal antibodies against troponin T with a cross-reactivity of < 2% against troponin I and other myofibrillary proteins, as well as of < 5% towards skeletal muscle troponin T by immunisation of experimental animals in combination with an adjuvant over several months with at least 4 immunisations, B-lymphocytes are obtained, these are fusioned with a permanent myeloma cell line, the clones isolated and the antibodies obtained herefrom.

## Revendications

1. Procédé pour la détermination de la nécrose des muscles cardiaques, basé sur le principe des dosages immunologiques, caractérisé en ce que l'on incube un échantillon de sérum ou de plasma avec au moins un anticorps dirigé contre la troponine T et un partenaire de liaison B pour la troponine T ou pour l'anticorps, soit l'anticorps dirigé contre la troponine T, soit le partenaire de liaison B, étant marqué avec un groupe détectable, on sépare l'immunocomplexe formé, qui contient le groupe détectable, et l'on détermine le groupe détectable dans la phase séparée ou dans la phase restante, comme mesure de la troponine T de l'échantillon.

2. Procédé selon la revendication 1, caractérisé en ce que l'on incube l'échantillon avec un anticorps dirigé contre la troponine T et un conjugué formé d'un anticorps dirigé contre la troponine T et d'un groupe détectable, on sépare l'immunocomplexe formé par séparation des phases et on détermine le groupe détectable dans l'une des phases.

3. Procédé selon la revendication 1, caractérisé en ce que l'on incube l'échantillon avec un anticorps dirigé contre la troponine T et un conjugué formé de la troponine T et d'un groupe détectable, on sépare l'immunocomplexe formé par séparation des phases et on détermine le groupe détectable dans l'une des phases.

4. Utilisation d'anticorps dirigés contre la troponine T pour la détermination de la nécrose des muscles cardiaques.

5. Anticorps monoclonal ou polyclonal dirigé contre la troponine T, ayant une réactivité croisée <2 % vis-à-vis de la troponine T et d'une autre protéine myofibrillaire ainsi qu'une réactivité croisée <5 % vis-à-vis de la troponine T des muscles du squelette.

6. Lignées cellulaires hybridomes ECACC 89060901 et ECACC 89030308.

7. Anticorps monoclonal préparé en utilisant les lignées cellulaires hybridomes ECACC 89060901 et ECACC 89030308.

8. Procédé de préparation d'anticorps polyclonaux dirigés contre la troponine T, ayant une réactivité croisée <2 % vis-à-vis de la troponine T et d'une autre protéine myofibrillaire ainsi qu'une réactivité croisée <5 % vis-à-vis de la troponine T des muscles du squelette, par immunisation d'animaux de laboratoire avec de la troponine T des muscles cardiaques humains, en combinaison avec un adjuvant pendant plusieurs mois, avec au moins 4 immunisations à des intervalles de quatre à six semaines, récupération du sérum brut et purification de ce dernier par immunosorption.

9. Procédé de préparation d'anticorps monoclonaux dirigés contre la troponine T, ayant une réactivité croisée <2 % vis-à-vis de la troponine T et d'une autre protéine myofibrillaire ainsi qu'une réactivité croisée <5 % vis-à-vis de la troponine T des muscles du squelette, par immunisation d'animaux de laboratoire avec de la troponine T des muscles cardiaques humains, en combinaison avec un adjuvant pendant plusieurs mois, avec au moins 4 immunisations à des intervalles de quatre à six semaines, récupération des lymphocytes B, fusion de ces derniers avec une lignée cellulaire myéloïde immortalisée, isolation des clones et à partir de là, récupération de l'anticorps.

Fig. 1

Fig. 2

Fig. 3